Europäisches Patentamt

(19) European Patent Office

Office européen des brevets

(11) Veröffentlichungsnummer: **0 009 138**
**B1**

(12) **EUROPÄISCHE PATENTSCHRIFT**

(45) Veröffentlichungstag der Patentschrift: **13.07.83**

(51) Int. Cl.³: **A 61 B 17/18**

(21) Anmeldenummer: **79103145.3**

(22) Anmeldetag: **27.08.79**

(54) Ein- und Ausschlagvorrichtung für Knochenmarknägel.

(30) Priorität: **04.09.78 AT 6367/78**

(43) Veröffentlichungstag der Anmeldung:
**02.04.80 Patentblatt 80/7**

(45) Bekanntmachung des Hinweises auf die Patenterteilung:
**13.07.83 Patentblatt 83/28**

(84) Benannte Vertragsstaaten:
**BE CH DE FR GB IT NL SE**

(56) Entgegenhaltungen:
**CH - A - 405 600**
**CH - A - 410 276**

(73) Patentinhaber: **METALLWERK PLANSEE GESELLSCHAFT M.B.H.**
**A-6600 Reutte, Tirol (AT)**
(73) Patentinhaber: **Otte, Wolf-Dieter**
**Am Model 14**
**D-8217 Volkach (DE)**

(72) Erfinder: **Otte, Heinz, Dr. med.**
**Am Model 14**
**D-8712 Volkach (DE)**
Erfinder: **Otte, Wolf-Dieter**
**Am Model 14**
**D-8712 Volkach (DE)**
Erfinder: **Schider, Siegfried, Dr.**
**Mittenwaldbahnstrasse 12**
**A-6600 Reutte-Breitenwang (AT)**
Erfinder: **Wiesner, Otto**
**Runzfeld 3**
**A-6600 Reutte-Mühl (AT)**

(74) Vertreter: **Lohnert, Wolfgang, Dr.**
**Metallwerk Plansee AG**
**A-6600 Reutte, Tirol (AT)**

Courier Press, Leamington Spa, England.

### Ein- und Ausschlagvorrichtung für Knochenmarknägel

Die Erfindung betrifft eine metallische Ein- und Ausschlagvorrichtung für Knochenmarknägel mit einem Führungsrohr und beidseitigen Anschlagelementen für eine auf dem Führungsrohr konzentrisch und verschiebbar angeordnete Schlagbüchse.

Bei einer aus der CH—A 410 276 bekannten Ein- und Ausschlagvorrichtung dieser Art erfolgt die Verbindung Führungsrohr — Knochenmarknagel mittels eines in beide Teile einschraubbaren Zwischenstiftes. Sein Durchmesser entspricht etwa dem Innendurchmesser des Nagels. Dieser kann daher bis unter die Knochenoberfläche eingetrieben werden. Da bei dieser Vorrichtung die Einschlagkraft direkt über die Gewindeverbindung übertragen wird, kann das Gewinde leicht durch Schlagkräfte beschädigt werden. Um eine Beschädigung des Gewindes in Grenzen zu halten, darf auch die von der Schlagbüchse erzielbare Schlagkraft nicht zu groß sein.

Der Erfindung lieft demegenüber die Aufgabe zugrunde, diese Ein- und Ausschlagvorrichtung für Knochenmarknägel so zu verbessern, daß Beschädigungen der Gewindeverbindung zwischen Knochenmarknagel und Führungsrohr der Ein- und Ausschlagvorrichtung durch Schlagkräfte vermieden werden und daß eine zumindest ebenso große oder gar größere Schlagkraft als bei der bekannten Ein- und Ausschlagvorrichtung aufgebracht werden kann, ohne daß die Schlagbüchse unhandliche Abmessungen aufweist. Die Eigenschaft der bekannten Ein- und Ausschlagvorrichtung, daß mit ihr ohne Verletzung des um das Knochenende herumliegenden Gewebes eingetrieben werden kann, soll dabei erhalten bleiben.

Diese Aufgabe wird erfindungsgemäß dadurch gelöst, daß das dem Knochenmarknagel zugeordnete Ende des Führungsrohres von zwei aufeinanderfolgenden zylindrischen Längsabschnitten, dem inneren Längsabschnitt und dem äußeren Längsabschnitt gebildet wird, daß sich die Rohrdurchmesser der einzelnen Längsabschnitte zum Nagelende hin gegenüber dem jeweils vorhergehenden Teil des Führungsrohres verringern und daß der dem Nagelende näherliegende Bereich des inneren Längsabschnittes mit einem Außengewinde versehen ist, daß weiterhin das dem Nagelende benachbarte Anschlagelement als Einschlaghülse ausgebildet ist, die auf dem inneren Längsabschnitt konzentrisch und verschiebbar angeordnet ist und deren Außendurchmesser etwa dem Durchmesser des Knochenmarknagels entspricht, und daß die Schlagbüchse eine spezifische Dichte größer als 10 g/cm$^3$ hat und ein Sinterverbundkörper, vornehmlich aus Wolfram mit Bindemittelzusätzen, oder ein mit Metallpulver oder -granulat gefüllter Stahlmantel ist.

Auf diese Weise wird erreicht, daß das Innengewinde des Knochenmarknagels beim Einschlagvorgang von den Einschlagkräften nicht belastet wird. Eine Beschädigung des Gewindes wird dadurch vermieden. Daher ist zum Herausziehen des Nagels nach erfolgtem Heilungsprozeß ein neuerliches Einschrauben des Führungsrohres in den Knochenmarknagel leicht möglich. Darüber hinaus kann, da die Gewindeverbindung von der Einschlagkraft nicht belastet wird, auch eine größere Einschlagkraft auf den Knochenmarknagel ausgeübt werden. Sie wird durch die Verwendung eines Materials hoher Dichte für die Schlagbüchse erreicht, auf Grund deren sich die gegenüber dem Stand der Technik vergrößerte Einschlagkraft ohne Vergrößerung, unter Umständen sogar unter einer Verkleinerung der Schlagbüchse und damit eine Verbesserung der Handlichkeit ergibt. Ein weiterer Vorteil der Erfindung besteht darin, daß die am inneren Längsabschnitt verschiebbare Einschlaghülse, die beim Einschlagvorgang auf dem Ende des Knochenmarknagels aufliegt, den bei nicht ganz eingeschraubtem Führungsrohr freiliegenden Gewindeabschnitt überdeckt, so daß ein Verklemmen oder Verletzen des Gewindes ausgeschlossen wird.

Bei einer bevorzugten Ausführungsform ist das am vom Knochenmarknagel abgewandten Ende des Führungsrohres angebrachte Anschlagelement abnehmbar und besteht aus einer zweiteiligen Hülse mit größerem Außendurchmesser als dem des Führungsrohres, die auf einen einen kleineren Durchmesser aufweisenden Längsabschnitt des Führungsrohres aufgesetzt und von einer über sie geschobenen Klemmhülse mit einem zur Schlagbüchse gerichteten Bund umschlossen ist.

Auf diese Weise wird eine gute Zerlegbarkeit und damit eine gute Sterilisierbarkeit der Ein- und Ausschlagvorrichtung erreicht.

Ein Ausführungsbeispiel der Erfindung wird nachfolgend an Hand der Figuren beschrieben. Es zeigt

Fig. 1 eine Ansicht der zusammengesetzten Ein- und Ausschlagvorrichtung und

Fig. 2 einen Schnitt durch das dem Knochenmarknagel abgewandte Ende des Führungsrohres mit Anschlagelement.

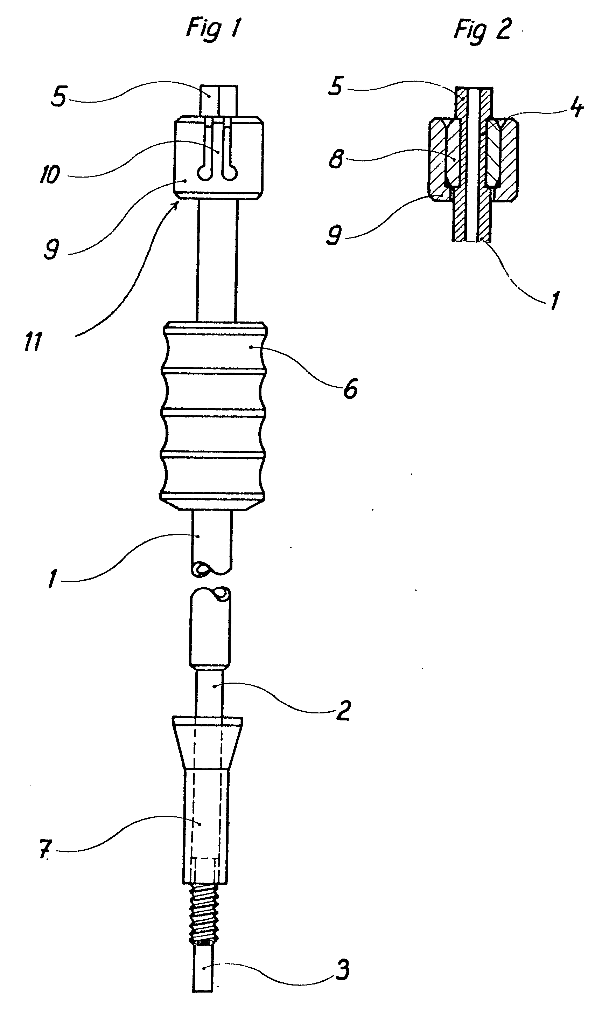

Fig. 1 zeigt das Führungsrohr 1, dessen Innendurchmesser so groß ist, daß es zusammen mit dem aufgeschraubten Knochenmarknagel über einen in den Knochen eingetriebenen und teilweise herausstehenden Führungsspieß geschoben werden kann. Der abgesetzte innere Längsabschnitt 2 des Führungsrohres 1, der an den abgesetzten äußeren Längsabschnitt 3 anschließt, ist etwas länger als die Einschlaghülse 7 ausgeführt und in seinem dem Knochenmarknagel näherliegenden Bereich mit einem Außengewinde versehen. Das Gewinde ist zum Anschrauben der Vorrichtung an den Knochenmarknagel vorgesehen. Der Durchmesser des äußeren Längsabschnittes 3 ist geringfügig kleiner als der Kerndurchmesser des Gewindes und dient als Führungsteil zum verkantungsfreien Einschrauben des Führungsrohres in den Knochenmarknagel. Auf dem inneren Längsabschnitt 2 ist die Einschlaghülse 7 konzentrisch und verschiebbar angeordnet; sie wird durch einen Sprengring am Abrutschen von dem Führungsrohr gehindert. Die Einschlaghülse 7 weist einen hohlzylinderförmigen Abschnitt auf, der in einen kegelstumpfförmigen Abschnitt mit zur Schlagbüchse hin zunehmendem Durchmesser übergeht. Der hohlzylinderförmige Abschnitt, dessen Durchmesser etwa dem des Knochenmarknagels entspricht, liegt beim Einschlagen mit seiner Stirnfläche auf der Stirnfläche des Knochenmarknagels auf. Dadurch, daß der Durchmesser des hohlzylinderförmigen Abschnittes etwa dem Durchmesser des Knochenmarknagels entspricht, kann der Nagel so weit eingeschlagen werden, daß die Einschlaghülse teilweise in den Knochen eindringt. Dadurch wird ein Vorstehen des Knochennagels über den Knochen vermieden. Die Stirnfläche des kegelstumpfförmigen Abschnittes wird mit einer auf dem Führungsrohr 1 konzentrisch und verschiebbar angeordneten Schlagbüchse 6, bestehend aus einem Sinterverbundkörper aus Wolfram und Bindemetall oder aus einem mit Wolframpulver gefüllten Stahlmantel, beaufschlagt.

Fig. 2 zeigt das dem Knochenmarknagel abgewandte Ende des Führungsrohres 1 mit einem auf einen kleineren Durchmesser abgesetzten Längsabschnitt 4, auf den eine zweiteilige Hülse 8 aufgesetzt ist, deren Außendurchmesser größer ist als der Außendurchmesser des Führungsrohres 1. Über die zweiteilige Hülse 8 ist eine Klemmhülse 9 mit einem zur Schlagbüchse 6 gerichteten und am Führungsrohr 1 anliegenden Bund geschoben.

Von einer federnden Zunge 10 wird die Klemmhülse 9 festgehalten. Diese abnehmbaren Teile dienen als Anschlagelement beim Ausschlagen des Knochenmarknagels; sie verhindern zugleich ein unbeabsichtigtes Gleiten der Schlagbüchse 6 über das Führungsrohr 1 hinaus beim Einschlagvorgang.

Durch die Ausbildung des dem Knochenmarknagel abgewandten Endes 5 des Führungsrohres 1 als Sechskant, auf welchen ein Schraubenschlüssel aufgesetzt werden kann, wird eine Drehung des Knochenmarknagels und damit eine bessere Positionierung während des Einschlagvorganges und eine bessere Lösung des Knochenmarknagels aus dem Markkanal während des Ausschlagvorganges ermöglicht.

Die Figuren geben eine mögliche Ausführungsform einer Ein- und Ausschlagvorrichtung wieder. Es ist auch denkbar, das Anschlagelement an dem dem Knochenmarknagel abgewandten Ende einteilig auszuführen und mit dem Führungsrohr zu verschrauben. Des weiteren kann dieses Anschlagelement ein stabförmiges, quer zum Führungsrohr verlaufendes Griffstück aufweisen, um eine bessere Handhabung der Ein- und Ausschlagvorrichtung zu gewährleisten. Aus demselben Grund ist est möglich, die Schlagbüchse mit einem drehbar gelagerten Handgriffstück zu versehen. Es ist auch möglich, für einen ersten Abschnitt des Einschlagvorganges die Einschlaghülse so auszubilden, daß das Ende des Knochenmarknagels bis zu einer als Auflage dienenden Schulter in die Einschlaghülse hineinragt. Dadurch ist das Nagelende von der Innenfläche des hohlzylindrischen Abschnittes umgeben, und eine Verformung durch das Einschlagen wird vermieden. Der letzte Abschnitt der Nagelung erfolgt dann mit einer Einschlaghülse der zuerst beschriebenen Art.

Ein weiterer Vorteil der Ein- und Ausschlagvorrichtung nach der Erfindung liegt darin, daß der äußere, abgesetzte Längsabschnitt des Führungsrohres, dessen Durchmesser geringfügig kleiner ist als der Kerndurchmesser des Gewindes des inneren Längsabschnittes, eine gute Führung beim Einschrauben des Führungsrohres in den Knochenmarknagel ergibt. Dadurch, daß das Absetzen auf kleinere Durchmesser nur über kleine Längsabschnitte erfolgt, ist eine genügende Festigkeit des Führungsrohres und damit eine gute Handhabung des gesamten Ein- und Ausschlagwerkzeuges gewährleistet.

Dadurch, daß die beiden Anschlagelemente und damit auch die Schlagbüchse 6 abnehmbar sind, wird eine gute Reinigung und Sterilisation des Ein- und Ausschlagwerkzeuges ermöglicht.

**Patentansprüche**

1. Metallische Ein- und Ausschlagvorrichtung für Knochenmarknägel mit einem Führungsrohr (1) und beidseitigen Anschlagelementen für eine auf dem Führungsrohr konzentrisch und verschiebbar angeordnete Schlagbüchse (6), dadurch gekennzeichnet, daß das dem Knochmarknagel zugeordnete Ende des Führungsrohres (1) von zwei aufeinanderfolgenden zylindrischen Längsabschnitten, dem inneren Längsabschnitt (2) und dem äußeren Längsabschnitt (3), gebildet wird, daß sich die Rohrdurchmesser der einzelnen Längsabschnitte zum Nagelende hin gegenüber dem jeweils vorhergehenden Teil des Führungsrohres verringern und daß der dem Nagelende näherliegende Bereich des inneren Längsabschnittes (2) mit einem Außengewinde versehen ist, daß weiterhin das dem Nagelende benachbarte Anschlagelement als Einschlaghülse (7) ausgebildet ist, die auf dem inneren Längsabschnitt (2) konzentrisch und verschiebbar angeordnet ist und deren Außendurchmesser etwa dem Durchmesser des Knochenmarknagels entspricht, und daß die Schlagbüchse eine spezifische Dichte größer als 10 g/cm³ hat und ein Sinterverbundkörper, vornehmlich aus Wolfram mit Bindemittelzusätzen, oder ein mit Metallpulver oder -granulat gefüllter Stahlmantel ist.

2. Metallische Ein- und Ausschlagvorrichtung nach Anspruch 1, dadurch gekennzeichnet, daß das am vom Knochenmarknagel abgewandten Ende des Führungsrohres (1) angebrachte Anschlagelement abnehmbar ist und aus einer zweiteiligen Hülse (8) mit größerem Außendurchmesser als dem des Führungsrohres (1) besteht, die auf einen einen kleineren Durchmesser aufweisenden Längsabschnitt (4) des Führungsrohres (1) aufgesetzt und von einer über sie geschobenen Klemmhülse (9) mit einem zur Schlagbüchse (6) gerichteten Bund umschlossen ist.

## Revendications

1. Dispositif métallique d'enfoncement et d'extraction pour des aiguilles à moëlle, comportant un tube de guidage (1) et des éléments de butée des deux côtés pour une douille de percussion (6) montée mobilement et concentriquement sur le tube de guidage, caractérisé par le fait que l'extrémité du tube de guidage (1) associée à l'aiguille à moëlle épinière est formée par deux sections longitudinales cylindriques successives, la section longitudinale intérieure (2) et la section longitudinale extérieure (3), que le diamètre tubulaire des sections longitudinales individuelles diminue en direction de l'extrémité de l'aiguille par rapport à la partie du tube du guidage respectivement située devant, et que la région de la section longitudinale intérieure (2) qui est la plus proche de l'extrémité de l'aiguille est munie d'un filetage extérieur, qu'en outre l'élément de butée voisin de l'extrémité de l'aiguille se présente sous la forme d'un manchan d'enfoncement (7) qui est monté de façon mobile et concentriquement sur la section longitudinale intérieure (2) et dont le diamètre extérieur correspond sensiblement au diamètre de l'aiguille à moëlle épinière, et que la douille de percussion possède une densité spécifique supérieure à 10 g/cm³ et est constituée par un corps composite fritté, principalement en tungstène avec des additifs de liaison, ou par une gaine d'acier remplie de poudre ou de granulat métallique.

2. Dispositif métallique d'enfoncement et d'extraction suivant la revendication 1, caractérisé par le fait que l'élément de butée disposé sur l'extrémité du tube de guidage (1) qui est opposée à l'aiguille à moëlle épinière peur être enlevé et est constitué par un manchon (8) en deux parties possédant un diamètre extérieur plus important que celui du tube de guidage (1), qui est monté sur une section longitudinale (4) du tube de guidage (1) possédant un diamètre plus faible, et est entouré par un manchon de serrage (9) enfilé par dessus et comportant un épaulement dirigé vers la douille de percussion (6).

## Claims

1. Metallic device for insertion and extraction of medullary nails with a guide tube (1) and striking elements on both ends for a concentrically and displaceable disposed strike bushing (6), characterized in, that the end of the guide tube (1) towards the medullary nail consists of two successive cylindric longitudinal sections, the inner longitudinal section (2) and the outer longitudinal section (3), that the tube diameters of the individual longitudinal sections diminishing in comparison to the respective preceding part of the guide tube and that the region of the inner longitudinal section (2) towards the end of the medullary nail being provided with an external thread, that further the striking element adjacent the end of the medullary nail is formed as in insertion sleeve (7), which is concentrically and displaceable disposed on the inner longitudinal section (2) and whose outside diameter is approximately equivalent to the diameter of the intramedullary nail and that the strike bushing whose specific density is greater than 10 g/cm³ is a sintered composite, principally of tungsten with binding metal additives or a metal powder or metal granulate enclosed in a steel jacket.

2. Metallic device for insertion and extraction according to claim 1, characterized in, that the striking element disposed at the end of the guide tube (1) farthest away from the medullary nail is removable and consists of a two-piece sleeve (8) of larger outside diameter than that of said guide tube (1), which is placed on a longitudinal section (4) of the guide tube (1) with a smaller diameter and which is enclosed in a clamping sleeve (9) slipped over said sleeve (8) with a collar directed to the strike bushing.

Fig 1

Fig 2